# NEUE EUROPÄISCHE PATENTSCHRIFT

(11) **EP 0 019 218 B2**
(45) Veröffentlichungstag und Bekanntmachung des Hinweises auf die Entscheidung über den Einspruch: **01.03.1995**
(45) Hinweis auf die Patenterteilung: 13.11.1985
(21) Anmeldenummer: 80102525.5
(22) Anmeldetag: 08.05.1980
(51) Int. Cl.: A61K 39/145, C12N 7/00, C12N 5/00

(54) **Influenzavirus-Vaccine, Verfahren zu ihrer Herstellung und Verfahren zur Vermehrung von Influenzaviren**
Influenza virus vaccines, process for preparing them and process for propagating influenza viruses
Vaccins antigrippaux, procédé pour leur préparation et procédé de propagation des virus de la grippe

(30) Priorität: 15.05.1979 US 39236
(43) Veröffentlichungstag der Anmeldung: 26.11.1980
(73) Patentinhaber: MOBAY CORPORATION, Pittsburgh Pennsylvania 15205-9741 (US)
(72) Erfinder: Brown, Karen K., Kansas City Missouri 64155 (US); Stewart, Richard C., Merriam Kansas 66203 (US)
(74) Vertreter: Schumacher, Günter, Dr.

(56) Entgegenhaltungen:
- FR-A- 2 262 962
- US-A- 0 033 164
- CHEMICAL ABSTRACTS, Band 84, Nr. 11, 15. März 1976, Seite 210 Zusammenfassung 71361p Columbus, Ohio, USA KLENK, Hans D. et al. "Activation of influenza A viruses by trypsin treatment"
- CHEMICAL ABSTARCTS, Band 84, Nr. 11, 15. März 1976, Seite 210 Zusammenfassung 71362q Columbus, Ohio, USA LAZAROWITZ, SOndra G. et al. "Enhancement of the infectivity of influenza A and B viruses by proteolytic cleavage of the hemagglutinin polypeptide"
- Virol. Arbeitsmethoden, Bd I, Gustav Fischer Verlag (1974), S. 231-233, 260-269.
- Japan, J.Med. Sci. Biol. 23, 227-235 (1970)
- Japan, J.Med. Sci., Biol. 23, 1-11 (1970)
- Bull, Wld.Hlth.Org.41 (1969)
- Virology 68, 426-439 (1975)
- Virology 68, 440-454 (1975)

## Beschreibung

Die Erfindung betrifft eine neue Influenzavirus-Vaccine sowie Verfahren zu ihrer Herstellung unter Verwendung einer mit Influenzaviren beimpften flüssigen Zellkultur.

Influenzavaccine sind seit den frühen Vierzigerjahren für die Impfung von Menschen und seit den späten Sechzigerjahren für die Impfung von Pferden in Gebrauch. Alle zur Zeit verwendeten Influenzavaccine werden durch Züchten der Vaccinevirusstämme im bebrüteten Hühnerei hergestellt. Die hierbei erhaltenen Virusstämme werden dann zur Herstellung von Virus-Lebendvaccinen verwendet oder zur Herstellung von abgetöteten Virusvaccinen weiterverarbeitet.

Es ist Virologen allgemein bekannt, daß Influenzaviren in Zellkulturen nur in sehr begrenztem Maße wachsen. Das Wachstum wird als »einstufiger Wachstumszyklus« bezeichnet, d. h. nur die ursprünglich infizierten Zellen pflanzen Viren fort. Diese Erscheinung wird beispielsweise von Davis et al. in MICROBIOLOGY, Harper and Row Publishers, Kapitel 44, S. 1138-1139 (1968) beschrieben. Da die Viren der ursprünglich infizierten Zellen aufeinanderfolgende Zahlen von Zellen in der gleichen Zellkultur nicht zu infizieren vermögen, sind die erhaltenen Ausbeuten bei weitem zu niedrig, um für die Herstellung von Virusvaccinen brauchbar zu sein. Flüssige Zellkulturen wurden daher für die Großherstellung von Influenzavirus-Vaccinen nicht verwendet.

Bebrütete Hühnereier werden zur Herstellung von Viren mit Titern verwendet, die für die Herstellung von Impfstoffen genügend hoch sind. Leider erfordern auf dem Brutei gezüchtete Viren gewöhnlich eine Konzentrierung und im Falle von menschlichen Vaccinen außerdem in irgendeiner Form eine Reinigung, um toxische Reaktionen als Folge des unerwünschten Eiereiweißes zu reduzieren. Die Verwendung von Eiern für die Impfstoffherstellung ist zeitraubend, arbeitsintensiv und erfordert verhältnismäßig hohe Materialkosten, und die Ausbeute aus einem Ei genügt gewöhnlich nur, um Vaccine für etwa 1 bis 1,5 Dosen herzustellen. Die Herstellung von Millionen Dosen erfordert somit die Beimpfung und Gewinnung von Millionen Bruteiern.

Vor kurzem wurde festgestellt, daß die verschiedensten Influenza-A-Viren einschließlich der bei Menschen, Pferden, Schweinen und Vögeln vorkommenden Stämme produktiv in einer festliegenden Linie von Hundenierenzellen unter einem Auflagemedium (»overlay medium«), das Trypsin enthält, wachsen und ohne Rücksicht auf ihre Passagenvorgeschichte wohldefinierte Plaques bilden; siehe die Arbeit von K. Tobita et al »Plaque Assay and Primary Isolation of Influenza A Viruses in an Established Line of Canine Kidnev Cells (MDCK) in the presence of Trypsin«, Med. Microbiol. Immunol. 162 (1975) 9-14. Ferner wird auf die Veröffentlichung von Hans-Dieter Klenk et al. »Activation of Influenza A Viruses by Trypsin Treatment« in Virology 68 (1975) 426-439 verwiesen. Es ist zu bemerken, daß bei den vorstehend genannten Berichten die Wirkungen von Trypsin auf die Influenzavirus-Vermehrung in halbfesten Kulturen bei Plaquebildungsbestimmungen und -isolierungsmethoden beobachtet wurden, bei denen es sich in keinem Fall um flüssige Zellkulturen oder die großtechnische oder kommerzielle Vermehrung von Influenzaviren für die Impfstoffherstellung handelte. Der hier gebrauchte Ausdruck »flüssige Zellkultur« bezeichnet die Züchtung von Zellen in vitro und die Vermehrung von Viren in einem chemisch definierten flüssigen Medium.

Es wurde nun überraschenderweise gefunden, daß proteolytische Enzyme ebenfalls in flüssigen Zellkulturen verwendet werden können, um die Infizierung aufeinanderfolgender Generationen von Zellen in der gleichen Zellkultur zu erleichtern. Dadurch, daß in dieser Weise die Begrenzungen des »einstufigen Wachstumszyklus« der bisherigen Methoden der Kultivierung in flüssigen Zellkulturen überwunden wurden, ist es möglich, eine Influenzavirus-Ausbeute zu erzielen, die 1000- bis 10 000fach größer ist als bei nicht mit Protease behandelten Kulturen. Dies ermöglicht die Anwendung der Methoden der Kultivierung in flüssigen Zellkulturen für die Großherstellung von Influenza-Impfstoffen, wodurch die mit der Verwendung von bebrüteten Hühnereiern verbundenen Nachteile vermieden werden. Einzelheiten des Kulturmediums, der Methoden der Virusvermehrung und der Vaccineherstellung sowie Gebrauchsmethoden werden nachstehend beschrieben.

In C. A. 84, (1976), 71361p und 71362q wird (ähnlich wie in den oben diskutierten Publikationen) die Verwendung von Trypsin zur Erhöhung der Infektivität von Influenzaviren bei der Plaquebildung beschrieben. Die ist jedoch nicht zu vergleichen mit der Verwendung von Trypsin (oder anderen Proteasen) zur Vermehrung von Grippeviren in flüssigen Zellkulturen und für die Herstellung von Impfstoffen. In den beiden Literaturstellen wird nichts über die Impfstoffherstellung ausgesagt. Sie betreffen vielmehr Untersuchungen zum Verständnis des Mechanismus der Virusinfektivität und der Erhöhung dieser Infektivität durch enzymatische Spaltung bestimmter Gruppen auf den Viren. Diese Erscheinung wird mittels der Plaquebildung verfolgt.

Die Plaquebildung deutet darauf hin, daß die untersuchten Viren eine nennenswerte Infektivität aufweisen. Solche Plaques werden in einem halbfester Medium (in der Regel Agar) untersucht.

Gemäß vorliegender Erfindung werden hingegen keine halbfesten Medien verwendet und auch keine Plaques gebildet. Vielmehr werden flüssige Zellkulturen zur Virusvermehrung eingesetzt. Gemäß Stand der Technik konnten derartige flüssige Zellkulturen für die Herstellung von Influenzavirus-Vaccinen nicht verwendet werden, da die Viren in solchen Kulturen keine nennenswerten Vermehrungsraten aufwiesen. Die erfindungsgemäße Mitverwendung von proteolytischen Enzymen ermöglicht nun erstmals die Verwendung flüssiger Zellkulturen zur Herstellung von Influenzavirus-Vaccinen im vorstehend angegebenen Maßstab.

Die vorliegende Erfindung betrifft somit ein Verfahren zur Herstellung einer Influenzavirus-Vaccine, die im wesentlichen frei ist von Eiereiweiß, wobei man Zellen mit einem Influenzavirus infiziert, das Virus in den Zellen vermehrt, danach das Virus isoliert und aus dem isolierten Virus eine Vaccine herstellt, welches dadurch gekennzeichnet ist, daß man einen Teil der Zellen einer flüssigen Zellkultur mit einem Influenzavirus infiziert und die infizierte Kulutur in Gegenwart von 4-25 µg/ml der flüssigen Zellkultur eines proteolytischen Enzyms inkubiert.

Das im erfindungsgemäßen Verfahren verwendete Influenzavirus-Vermehrungsmedium umfaßt eine Zellkultur, die mit einem Influenzavirus infizierbar ist, ein Influenzavirus und ein proteinhydrolysierendes Enzym, das in einer Konzentration von 4-25 µg/ml der flüssigen Zellkultur vorliegt. Das Verfahren gemäß der Erfindung umfaßt die Stufen der Beimpfung oder Infizierung einer flüssigen Influenzavirus-Zellkultur mit den Infuenza-Viren, der Inkubation des Inoculats in Gegenwart von 4-25 µg/ml der flüssigen Zellkultur eines proteinhydrolysierenden Enzyms unter Bedingungen, die genügen, um das in den Beispielen demonstrierte Viruswachstum (oder einen entsprechenden zytopathischen Effekt) zu gewährleisten, und der Gewinnung des Virus. Die Infizierung der Zellen mit dem Virus kann vor oder nach der Bildung der Monoschicht der Zellen oder auch einfach durch Infizierung einer flüssigen Suspension der Zellen.

In einer anschließenden Stufe wird das geerntete Virus abgetötet oder durch eine weitere Passage in der Zellkultur für den Vaccinegebrauch abgeschwächt. Bei besonders bevorzugten Ausführungsformen wird die Protease Trypsin in Verbindung mit einer Hundenierenzellinie zur Vermehrung aller beliebigen verschiedenen Typen von Influenza-Viren verwendet.

Die Erfindung wird nachstehend unter Bezugnahme auf die Abbildungen weiter erläutert.

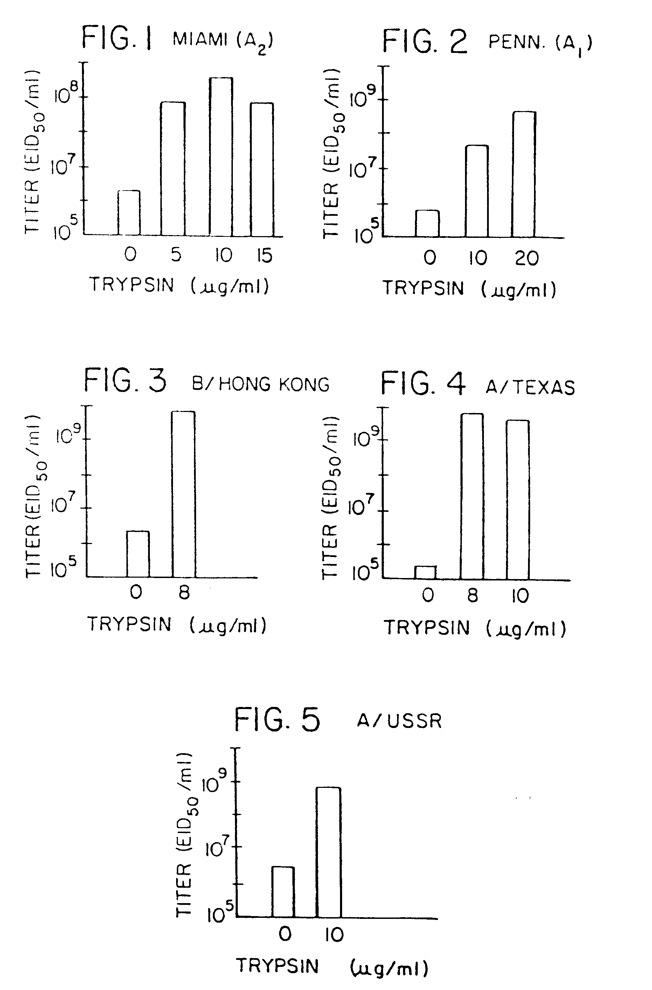

Fig. 1 bis 5 sind Balkendiagramme, die die Steigerungen des Titers veranschaulichen, die erreicht wurden, wenn die genannten Virusstämme in einer flüssigen Zellkultur in Gegenwart verschiedener Mengen einer typischen Protease, des Trypsins, inkubiert wurden. Die Ergebnisse sind als geometrische mittlere Titer angegeben. Nachstehend werden spezielle Ausführungsformen der Erfindung beschrieben.

Im erfindungsgemäßen Verfahren ist die Verwendung von Influenza-Vaccineviren vorgesehen. Der hier gebrauchte Ausdruck »Influenzavirus« umfaßt alle Viren, die in der Lage sind, bei Tieren (einschließlich des Menschen) einen febrilen Krankheitszustand hervorzurufen, der durch respiratorische Symptome, Schleimhautentzündung und häufig systematischen Befall gekennzeichnet ist. Das erfindungsgemäße Verfahren ist besonders vorteilhaft für die Erzeugung der verschiedensten Influenza-Viren einschließlich der Stämme, die Mensch, Pferd, Schwein und Vögel befallen. Beispiele der Herstellung typischer Influenza-Viren des Typs A und B werden später beschrieben.

Die Impfstoffe gemäß der Erfindung umfassen von Eiereiweiß freie Präparate von abgetöteten, lebenden abgeschwächten oder lebenden voll virulenten Influenza-Viren, die verabreicht werden können, um Immunität gegen jede Influenza-Erkrankung zu erzeugen oder künstlich zu steigern. Bei bevorzugten Ausführungsformen liegt die Vaccine als wäßrige Suspension der Viruspartikel in gebrauchsfertiger Form vor.

Für die Zellkulturen, die als geeignet für die Durchführung des Verfahrens gemäß der Erfindung vorgesehen sind, kommen alle tierischen Zellstämme oder -linien infrage, die durch einen oder mehrere gegebene Influenza-Virusstämme infizierbar sind und die Vermehrung dieser Virusstämme ermöglichen. Zwar ist eine Anzahl solcher Zellen bekannt und wird als geeignet für die hier beschriebenen Verfahren gehalten, jedoch wurden von der Anmelderin besonders gute Ergebnisse mit einem ganz bestimmten Zellstamm erhalten, der als Cutter Laboratories Dog Kidney (CLDK)-Zellstamm (Hundenieren-Zellstamm oder Cutter Laboratories) bekannt ist. Der CLDK-Zellstamm ist vom Landwirtschaftsministerium der USA für die Verwendung zur Herstellung von Veterinär-Vaccinen genehmigt worden und ist dem Madin Darby-Hundenieren-Zellstamm (ATCC Nr. CCL 34) und den in der US-PS 3 616 203 beschriebenen Hundenieren-Zellstamm ähnlich. Nachstehend folgen eine kurze Geschichte und eine Beschreibung des für den Zellstamm in den Beispielen verwendeten speziellen Ursprungs-Gewebematerials, obwohl natürlich die hier beschriebenen Methoden als geeignet für alle für Influenza-Viren brauchbaren Zellkulturen gehalten werden.

### Hundenieren-Zellstamm der Cutter Laboratories (CLDK)

### Geschichte

Der Ursprungsstamm von CLDK wurde von der Niere eines offensichtlich normalen Beagle-Hundes, der von der Universität California in Davis erhalten worden war, durch die Cutter Laboratories, Inc., Berkeley, California (USA), herangezüchtet und als Permanentkultur weitergeführt. Der Stamm wurde auf 0,5% Milcheiweißhydrolysat und 0,2% Hefeextrakt in Earle's ausgewogener Salzlösung plus 5% Kalbs-, Schafs- oder Pferdeserum und Antibiotika gehalten und nach den Methoden von J. S. Younger (Proc. of Exp. Biol. and Med., V 85,202) kultiviert.

Eine eingefrorene Ampulle der 142. Passage dieses Zellstamms wurde anschließend in einem 75 cm² (250 ml)-Falconkolben in ein Gewebekulturmedium gegeben, das aus Earle's ausgewogener Salzlösung als essentielles Minimum-Medium (Earle's balanced salt solution Minimum Essential Medium (MEM)) und 10% fötalem Rinderserum bestand. Die Zellen wurden in der gleichen Weise und im gleichen Medium weitergezüchtet, um das eingefrorene Masterzellmaterial in der Passage 148 herzustellen.

Eine Ampulle des Masterzellmaterials wurde aufgetaut und reihenmäßig 20mal weiterkultiviert, wobei Flaschenkulturen der 168. Passage erhalten wurden. Diese Zellen wurden dann eingefroren.

### Beschreibung des Masterzellmaterials (MCS)

Nummer der Seriensubkulturen vom Ursprungsgewebe: 148
Gefriermedium:
Essentielles Mindestmedium (Eagle) in Earle's BSS mit reduziertem Bicarbonat (1,65 g/l) 80%;
fötales Rinderserum 10%; Dimethylsulfoxid 10%.
Lebensfähigkeit:
Ungefähr 75% (Farbstoffausschluß).
Kulturmedium:
Essentielles Mindestmedium (minimum essential medium) (Eagle) in Earle's BSS mit reduziertem Bicarbonat (1,65 g/l) 90%; fötales Rinderserum 2-10%; Antibiotika: Penicillin und Streptomycin 100 E. oder /ml.
Wachstumsmerkmale der aufgetauten Zellen:
Ein Inoculum von 3 x 10⁶ lebensfähigen Zellen/ml, kultiviert im vorstehend genannten Kulturmedium bei 37°C in einem geschlossenen System, vermehrt sich in 5 Tagen ungefähr 6- bis 8fach.
Morphologie:
epithelialartig.
Karyologie:
Chromosomenfrequenzverteilung 100 Zellen: 2N = 72.

| | | | | | | | | | |
|---|---|---|---|---|---|---|---|---|---|
| Zellen: | 1 | 1 | 2 | 4 | 6 | 9 | 69 | 5 | 3 |
| Chromosomen: | 60 | 65 | 68 | 69 | 70 | 71 | 72 | 73 | 74 |

### Keine Markierungschromosomen.

Sterilitätstests:
Frei von Mykoplasma, Bakterien und Pilzen
Spezies:
Durch Immunofluoreszenztest als Hund bestätigt.
Virus-Anfälligkeit:
Empfänglich für Influenza-Viren, Rabies-Virus, infektiöse Rinder-Rhinotracheitis, infektiöse Hundehäpatitis, Hundestaupe-Virus und möglicherweise andere Viren.

Zu den Proteine hydrolisierenden Enzymen (proteolytisches Enzym oder Protease), die als geeignet für die Zwecke der Erfindung vorgesehen sind, gehören bekannte Proteasen, z. B. Trypsin, Chymotrypsin, Pepsin, Pancreation, Papain, Pronase, Carboxypeptidase u. dgl., wobei Trypsin ein besonders bevorzugtes Enzym ist. Der genaue Mechanismus bzw. die genauen Mechanismen, nach denen eine Protease wie Trypsin die Influenzavirus-Infektiosität steigert, sind noch nicht völlig geklärt. Ein möglicher Mechanismus wird in dem vorstehend genannten Artikel von Klenk et al vorgeschlagen.

Wie nachstehend beschrieben, sollte die Menge der aktiven Protease, die erforderlich ist, um die aufeinanderfolgende Infektuosität zu steigern, wenigstens genügen, um die Begrenzungen des einstufigen Wachstumszyklus auszuschalten, aber im Falle von konfluierenden Monoschichtkulturen nicht so hoch sein, daß Ablösung von konfluierenden Zellen von den Oberflächen des Gewebekulturgefäßes (d. h. der Innenfläche einer Rollflasche) verursacht wird. Im Falle des bei den in den folgenden Beispielen beschriebenen Versuchen verwendeten speziellen Enzyms wird eine Menge des Enzyms im Bereich von etwa 4 bis 25 µg/ml des flüssigen Gewebekulturmediums, insbesondere eine Menge von etwa 10 µg/ml, bevorzugt.

### Virusvermehrungsmethoden

Das hier beschriebene Verfahren zur Vermehrung von Influenza-Viren umfaßt die drei allgemeinen Stufen der Infizierung eines Teils der Zellen einer flüssigen Zellkultur mit dem Influenza-Virus, der Inkubation der Zellen in Gegenwart eines proteolytischen Enzyms unter Bedingungen, die genügen, um maximalen zytopathischen (CP) Effekt zu gewährleisten, und der Isolierung der Viren von der Kultur. Die Impfstoffherstellung umfaßt die anschließende Stufe der Modifizierung des isolierten Virus nach bekannten Methoden zur Herstellung einer Lebendvaccine mit virulenten oder abgeschwächten Viren oder einer Vaccine mit abgetöteten (inaktiven) Viren. Die Vaccine kann in trockener Form für die Vermischung mit einem Verdünnungsmittel oder in flüssiger, gebrauchsfertiger Form vorliegen. Wie nachstehend beschrieben, können geeignete Adjuvantien zur Steigerung der Immunisierungsfähigkeit einbezogen werden.

Die Protease kann einer wäßrigen Suspension der Zellkultur oder vor oder nach der Bildung einer konfluenten Monoschichtkultur zugesetzt werden. Einige der verschiedenen Vermehrungsmethoden werden nachstehend als Beispiel beschrieben.

### Methode A

### Infizierung der vorgebildeten Monoschichten

1. Die Zellen werden bis zur Konfluenz in Kulturgefäßen wie Rollflaschen, Povitsky-Kolben oder Roux-Flaschen unter Verwendung bekannter Zellkulturmedien gezüchtet.
2. Vor der Infizierung wird das Nährmedium aus den Monoschichten der Zellen entfernt.
3. Das Arbeits-Impfmaterial des Influenza-Virus wird im Züchtungsmedium verdünnt, das zusätzliche Vitamine, nicht-essentielle Aminosäuren, L-Glutamin, Dextrose und Antibiotika anthält, wobei der pH-Wert auf 6,6 bis 6,8 eingestellt wird.
4. Eine Menge des das Virus enthaltenden Verdünnungsmittels wird zur Zellmonoschicht in Mengen von 10% bis 100% des endgültigen, für die Isolierung vorgesehenen Volumens gegeben.
5. Die infizierten Monoschichten werden 1 bis 72 Stunden bei 34°C bis 37°C inkubiert.
6. Protease wird allein oder in Kombination mit dem Viruszüchtungsmedium in einer Konzentration zugesetzt, die das Wachstum in vielfachen Zyklen fördert, ohne Löslösung von Zellen hervorzurufen. Bei Trypsin liegt diese optimale Konzentration zwischen etwa 8 und 15 µg/ml.
7. Die Viruszüchtungsbehälter werden erneut bei 34° bis 37°C inkubiert, bis maximale zytopathische Effekte beobachtet werden. Zu diesem Zeitpunkt werden die Virusflüssigkeiten isoliert.
8. Zur Isolierung werden die Behälter kräftig geschüttelt, um die Zellen zu entfernen, und die Flüssigkeiten und Zellen in einen sterilen Behälter zur weiteren Verarbeitung überführt.

### Methode B

### Infizierung der flüssigen Zellsuspension vor der Monoschichtbildung

1. Die Zellen werden aus den Kultivierungsbehältern nach üblichen Verfahren entfernt.
2. Die Zellen werden durch Zentrifugation eingeengt und dann in einer Menge frischen Nährmediums, das zusätzliche Vitamine, nicht-essentielle Aminosäuren, L-Glutamin, Dextrose und Antibiotika enthält, erneut suspendiert.
3. Dieser konzentrierten Zellsuspension wird dann Influenzavirus zugesetzt.
4. Die Zell-Virus-Suspension wird in einem sterilen geschlossenen Gefäß (z. B. einem Erlenmeyerkolben mit Schraubkappe) inkubiert (25° bis 37°C), während sie auf einem Magnetrührer oder einer rotierenden Schüttelvorrichtung 10 Minuten bis 4 Stunden gemischt wird.
5. Aliquote Teile der Zell-Virus-Suspension werden in Züchtungsbehälter (Rollflaschen, Roux-Flaschen, Povitsky-Kolben) gegeben, wobei das volle Volumen der Medien die in B-2 genannten Bestandteile plus 5% fötales Kalbserum enthält.
6. Die Kultivierungsbehälter werden bei 34° bis 37°C inkubiert, bis konfluente Monoschichten sich gebildet haben (ungefähr 2 bis 4 Tage).
7. Nachdem sich Monoschichten gebildet haben, wird Protease in einer Konzentration zugesetzt, die Wachstum in vielen Zyklen fördert, ohne daß Zellen abgelöst werden. Bei Trypsin liegt diese optimale Konzentration zwischen 10 und 25 µg/ml.
8. Die Kultivierungsbehälter werden bei 34° bis 37°C inkubiert, bis maximale zytopathische Effekte beobachtet werden. Das Virus wird dann isoliert.
9. Zur Isolierung werden die Behälter kräftig geschüttelt, um die Zellen zu entfernen, und die Zellen und Flüssigkeiten in einen sterilen Behälter zur weiteren Verarbeitung überführt.

### Methode C

### Beimpfen einer Kultur in flüssiger Suspension

1. An die Suspensionskultur angepaßte Zellen werden bis zur optimalen Zahl im Nährmedium in Behältern für die Suspensionskultur gezüchtet.
2. Die Zellen werden zentrifugiert und in einer Menge frischen Nährmediums, das zusätzliche Vitamine, nicht essentielle Aminosäure, L-Glutamin, Dextrose und Antibiotika enthält, erneut suspendiert.
3. Influenzavirus wird dann zugesetzt und das Kulturmedium bei 34° bis 37°C 10 Minuten bis zu einigen Stunden inkubiert.
4. Fötales Kalbsserum kann zugesetzt werden, und die Kultursuspension wird 1 bis 72 Stunden bei 34° bis 37°C weiter inkubiert.
5. Protease wird in einer Konzentration zugesetzt, die Züchtung in vielen Zyklen ermöglicht, ohne daß eine nachteilige Wirkung auf die Zellen ausgeübt wird. Bei Trypsin liegt die optimale Konzentration zwischen 4 und 25 µg/ml.
6. Die Inkubation bei 34° bis 37°C wird fortgesetzt, bis maximale zytopathische Effekte beobachtet werden. Zu diesem Zeitpunkt werden die Flüssigkeiten isoliert.
7. Zur Isolierung werden die Flüssigkeiten in einen sterilen Behälter zur weiteren Verarbeitung überführt.

Nachstehend folgen spezielle Beispiele der Anwendung der Methoden gemäß der Erfindung und der Nährmedien für die Vermehrung ausgewählter Stämme von Influenzaviren. Falls nicht anders angegeben, wurden übliche bekannte Gewebekulturverfahren angewendet. Da die Verfahren der Herstellung und Vorbereitung sowohl der Zellen als auch des Mediums bekannt sind, werden sie hier nicht ausführlich beschrieben.

### Beispiel 1

Das Pferde-Influenzavirus A2, als Miami-Stamm bezeichnet, wurde ursprünglich von einem Pferd an der Universität Miami isoliert. Dieses Virus wurde von der Medical School der Universität Pennsylvania bezogen, wo es in 6 Passagen über bebrütete Hühnereier geführt wurde. Die 7. Passage wurde bei Lederle Laboratories durchgeführt und von diesen erhalten. Der Stamm wurde über weitere Passagen in bebrüteten Hühnereiern geführt und in den Passagen 11 bis 16 verwendet. Das zur Zeit bevorzugte Verfahren zur Vermehrung des Stamms A2 in der Gewebekultur wird wie folgt durchgeführt: Eine junge, konfluente Monoschicht von CLDK-Zellen wird beimpft. Die Zellen werden in Rollflaschen eingesetzt, wobei Hank's essentielles Mindestmedium (Minimum Essential Medium) (MEMH), das die folgenden Bestandteile enthält, verwendet wird:

| | |
|---|---|
| Fötales Rinderserum | 5 bis 10% |
| nicht-essentielle Aminosäuren | 10 ml/l (Gibco) |
| L-Glutamin | 10 ml/l (Gibco) |
| Neomycinsulfat | 30 000 µg/l |
| Polymyxin B | 30 000 Einheiten/l |
| Mycostatin | 25 000 Einheiten/l |

Die Zellen sind gewöhnlich nach 72 Stunden konfluent. Zu diesen Zeitpunkt wird das Medium abgegossen, und die Zellen werden infiziert. Das Impfmedium enthält das A2-Virus auf einen »Egg Infective Dose₅₀(EID₅₀)«-Titer von etwa 10^{3,0}/ml in MEMH verdünnt, das mit den folgenden Bestandteilen ergänzt ist:

| | |
|---|---|
| 50%ige Dextrose | 2,6 ml/l |
| MEM-Vitamine | 30ml/l (Gibco) |
| nicht essentielle Aminosäuren | 10 ml/l (Gibco) |
| L-Glutamin | 10 ml/l (Gibco) |
| Neomycinsulfat | 30 000 µg/l |
| Polymyxin B | 30 000 Einheiten/l |
| Mycostatin | 25 000 Einheiten/l |

Das Inoculierungsmedium, das 14% des Endvolumens entspricht, wird zu jeder Rollflasche gegeben, und die Behälter werden 72 Stunden bei 34 bis 35°C inkubiert. Zu diesem Zeitpunkt wird das verbliebene Medium (86%), das 12 µg/ml sterile Trypsinlösung (Sigma 1 : 250) (0,1 g/100 ml) enthält, zu jeder Rollflasche gegeben. Die Gefäße werden erneut bei 34 bis 35°C inkubiert, bis der maximale zytopatische Effekt beobachtet wird (48 bis 72 Stunden). Zu diesem Zeitpunkt werden die Flüssigkeiten isoliert. Zur Isolierung werden die Rollflaschen zur Entfernung von etwa anhaftenden Zellen kräftig geschüttelt und die Zellen und Virusflüssigkeiten zur Weiterverarbeitung in einen sterilen, chargenweise arbeitenden Behälter überführt.

Die EID₅₀-Titer des nach diesem Verfahren gezüchteten Influenzavirus A2 nach der Isolierung sind in Tabelle 1 genannt. Ferner wird auf Fig. 1 verwiesen. Ein Vergleich wird mit dem nach dem gleichen Verfahren, jedoch ohne Zusatz von Trypsin gezüchteten A2-Virus vorgenommen.

**Tabelle 1**

| EID₅₀ von Pferde-Influenzavirus, Miamistamm, gezüchtet in CLDK-Zellen mit und ohne Zusatz von Trypsin | | | |
|---|---|---|---|
| Trypsinmenge, µg/ml | Titer (EID₅₀/ml) Einsatz | nach Isolierung | x-fache Steigerung mit Trypsin |
| - | 10^{2,6} | 10^{6,2} | - |
| - | 10^{3,2} | 10^{6,2} | - |
| 5 | 10^{2,9} | 10^{8,3} | 126 |
| 5 | 10^{2,9} | 10^{8,1} | 79 |
| 5 | 10^{2.6} | > 10^{9,2} | > 1000 |
| 10 | 10^{2,9} | 10^{9,2} | 1000 |
| 10 | 10^{2,9} | 10^{8,5} | 200 |
| 10 | 10^{3,2} | 10^{8,2} | 100 |
| 10 | 10^{3,2} | 10^{10,0} | 6310 |
| 10 | 10^{3,1} | 10^{14,5} | 199, 526, 232 |
| 15 | 10^{3,1} | 10^{8,9} | 501 |

Die Zugabe von Trypsin zum Kulturmedium ergab einen geometrischen Anstieg von 3,2 log oder 1711mal so viele Viruspartikel/ml während der Produktion des Miami-Stamms.

### Beispiel 2

Eine Probe des virulenten Typs des Pferde-Influenzavirus A1 wurde von der Medical School der Universität Pennsylvania bezogen. Der als Pennsylvania (A1) bezeichnete Stamm wurde von einem Pferd isoliert und über sechs Passagen im bebrüteten Hühnerei geführt. Der Stamm wurde über weitere Passagen im Brutei geführt und wird bei den Passagen 12 bis 17 für die Gewebekulturproduktion verwendet.

Für die Vermehrung des Stamms A1 in der Gewebekultur wird die folgende Methode bevorzugt: Eine Suspension von CLDK-Zellen wird vor der Bildung der Monoschicht infiziert. Die CLDK-Zellen in einer Konzentration von etwa 10^{5,5}/ml, Pennsylvania-Stamm des Virus bei einem EID₅₀-Titer von 10^{3,0}/ml bis 10^{5,0}/ml und Hank's Minimum Essential Medium (MEMH), das durch die nachstehend genannten Stoffe ergänzt ist, werden bei 25°C inkubiert, währens sie auf einem Magnetrührer in einem geschlossenen sterilen Erlenmeyerkolben gemischt werden. Der pH-Wert wird während der Inkubationszeit von 2 bis 3 Stunden mit 1N-HCl bei 6,7-6,8 gehalten.

| Ergänztes MEMH-Medium | |
|---|---|
| 50%ige Dextrose | 2,6 ml/l |
| MEM-Vitamine | 30 ml/l (Gibco) |
| Nicht-essentielle Aminosäuren | 10 ml/l (Gibco) |
| L-Glutamin | 10 ml/l (Gibco) |
| Neomycinsulfat | 30 000 µg/l |
| Polymyxin B | 30 000 Einheiten/l |
| Mycostatin | 25 000 Einheiten/l |

Nach dieser Züchtung in Suspension werden aliquote Teile von je 10 ml der Zell-Virus-Suspension in Rollflaschen gegeben, die 11 des in der vorstehend genannten Weise ergänzten MEMH-Mediums, das 5% fötales Kalbsserum enthält, enthalten. Die Rollflaschen werden bei 34° bis 35°C inkubiert, bis die Monoschicht konfluent ist (ungefähr 48 bis 72 Stunden), worauf 20 ml einer sterilen Trypsinlösung von 1 mg/ml (Sigma 1 : 250) in jede Rollflasche gegeben werden, Die Rollflaschen werden erneut bei 34° bis 35°C inkubiert, bis der maximale zytopathische Effekt beobachtet wird (3 bis 5 Tage). Die Virusflüssigkeiten werden isoliert, indem jede Rollflasche zur Entfernung der haften gebliebenen Zellen kräftig geschüttelt wird und die Zellen und Flüssigkeiten zur weiteren Verarbeitung in ein steriles Gefäß überführt werden.

Die EID₅₀-Titer des nach diesem Verfahren gezüchteten Influenzavirus A1 sind in Tabelle 2 genannt. Ferner wird auf Fig. 2 verwiesen. Ein Vergleich wird mit dem nach dem gleichen Verfahren ohne Trypsin gezüchteten Virus A1 vorgenommen.

**Tabelle 2**

| EID₅₀-Titer von in CLDK-Zellkultur mit und ohne Trypsin gezüchtetem Pferde-Influenzavirus, Stamm Pennsylvania | | | |
|---|---|---|---|
| Trypsinmenge, µg/ml | Titer (EID₅₀/ml) Einsatz | nach Isolierung | x-fache Steigerung mit Trypsin |
| - | 10^{5,3} | 10^{5,9} | - |
| - | 10^{4,9} | 10^{5,4} | - |
| 10 | 10^{5,3} | 10^{7,4} | 50 |
| 10 | 10^{4,9} | 10^{6,8} | 13 |
| 10 | 10^{5,1} | 10^{8,0} | 200 |
| 20 | 10^{5,3} | 10^{8,7} | 1000 |
| 20 | 10^{4,9} | 10^{8,5} | 631 |
| 20 | 10^{5,0} | 10^{7,1} | 25 |
| 20 | 10^{4,9} | 10^{8,3} | 398 |
| 20 | 10^{3,2} | 10^{9,2} | 3162 |
| 20 | 10^{3,2} | 10^{7,5} | 63 |
| 20 | 10^{3,2} | 10^{8,2} | 316 |

Die Zugabe von Trypsin zum Kulturmedium ergab einen mittleren geometrischen Anstieg von 2,3 log oder 187mal so viele Viruspartikel/ml während der Produktion des Pennsylvania-Stamms.

### Beispiel 3

Ein Stamm des menschlichen Influenzavirus mit der Bezeichnung B/Hongkong/5/72 (BX-1) wurde vom The Center for Disease Control in Atlanta, Georgia, bezogen. Dieser Stamm wurde in einer Passage über bebrütete Hühnereier geführt und bei -70°C als Arbeitsimpfvirus eingefroren.

Für die Vermehrung des Stamms B/Hongkong/5/72 in der Gewebekultur wird die folgende Methode bevorzugt: Eine junge konfluente Monoschicht von CLDK-Zellen ähnlich denen in Beispiel 1 wird infiziert. Die Zellen werden auf die in Beispiel 1 beschriebene Weise gezüchtet. Das Nährmedium wird von den Zellen entfernt und verworfen. Die Zellen werden dann mit dem Virus infiziert, das in dem in Beispiel 1 genannten Impfmedium auf einen EID₅₀-Titer von etwa 10^{3,0-5,0}/ml verdünnt worden ist. Das Inoculum besteht aus einem Volumen, das 33,3% des endgültigen isolierten Volumens entspricht. Gefäße werden 40 bis 48 Stunden bei 34° bis 35°C inkubiert, worauf das restliche Medium (66,7%), das 12 µg/ml Trypsinlösung (0,1 g/100 ml) enthält, jedem Gefäß zugesetzt wird. Die Inkubation bei 34° -35°C wird fortgesetzt, bis der maximale zytopathische Effekt beobachtet wird (48 bis 72 Stunden), worauf die Virusflüssigkeiten isoliert werden. Die Isolierung erfolgt durch kräftiges Schütteln jedes Gefäßes zur Entfernung der Zellen und Überführung von Zellen und Flüssigkeiten in ein steriles Gefäß zur Weiterverarbeitung.

Die EID₅₀-Titer des nach diesem Verfahren gezüchteten Influenzavirus B/Hongkong/5/72 nach der Isolierung sind nachstehend in Tabelle 3 genannt. Ferner wird auf Fig. 3 verwiesen. Ein Vergleich wird mit diesem nach dem gleichen Verfahren, jedoch ohne Zusatz von Trypsin gezüchteten Virus vorgenommen.

**Tabelle 3**

| EID₅₀-Titer des menschlichen Influenzavirusstamms B/Hong Kong/5/72, der in CLDK-Zellen mit und ohne Trypsin gezüchtet worden ist | | | |
|---|---|---|---|
| Trypsinmenge, µg/ml | Titer (EID₅₀/ml) Einsatz | nach Isolierung | x-fache Steigerung mit Trypsin |
| - | 10^{3,2} | 10^{6,0} | - |
| - | 10^{5,7} | 10^{6,4} | - |
| 8 | 10^{3,0} | 10^{8,7} | 316 |
| 8 | 10^{2,0} | 10^{9,0} | 631 |
| 8 | 10^{4,5} | 10^{9,5} | 1995 |
| 8 | 10^{3,5} | > 10^{10,2} | > 10 000 |

Die Zugabe von Trypsin zum Kulturmedium ergibt einen geometrischen mittleren Anstieg von 3,1 log oder 1412mal so viele Viruspartikel während der Produktion des Stamms B/Hong Kong.

### Beispiel 4

Ein Stamm des menschlichen Influenzavirus mit der Bezeichnung A/Texas/1/77 wurde von The Center for Disease Control in Atlanta, Georgia, bezogen. Dieser Stamm wurde in einer Passage über bebrütete Hühnereier geführt und bei -70°C als Arbeitsimpfvirus eingefroren.

Für die Vermehrung des Stamms A/Texas/1/77 in der Gewebekultur wird das insgesamt in Beispiel 3 beschriebene Verfahren bevorzugt.

Die EID₅₀-Titer des nach diesem Verfahren gezüchteten und isolierten menschlichen Influenzavirus-Stamms A/Texas/1/77 sind in Tabelle 4 genannt. Ferner wird auf Fig. 4 verwiesen. Ein Vergleich wird mit diesen nach dem gleichen Verfahren, jedoch ohne Zusatz von Trypsin gezüchteten Stamm vorgenommen.

**Tabelle 4**

| EID₅₀-Titer des in CLDK-Zellen mit und ohne Trypsin gezüchteten menschlichen Influenzavirus, Stamm A/Texas/1/77 | | | |
|---|---|---|---|
| Trypsinmenge, µg/ml | Titer (EID₅₀/ml) Einsatz | nach Isolierung | x-fache Steigerung mit Trypsin |
| - | - | 10^{5,2} | - |
| 8 | 10^{5,1} | 10^{8,9} | 5,012 |
| 8 | 10^{4,1} | >10^{10,2} | > 100 000 |
| 10 | 10^{3,0} | 10^{8,3} | 1259 |
| 10 | 10^{2,0} | 10^{9,8} | 39 811 |

Die Zugabe von Trypsin zum Kulturmedium ergab einen geometrischen mittleren Anstieg von 4,1 log oder 12 590mal so viele Viruspartikel während der Produktion des A/Texas-Stamms.

### Beispiel 5

Ein Stamm des menschlichen Influenzavirus mit der Bezeichnung A/USSR/90/77 wurde von The Center for Disease Control in Atlanta, Georgia, bezogen. Dieser Stamm wurde mit einer Passage über bebrütete Hühnereier geführt und bei -70°C als Arbeitsimpfvirus eingefroren.

Für die Vermehrung des Stamms A/USSR/90/77 in der Gewebekultur wird das insgesamt in Beispiel 3 beschriebene Verfahren bevorzugt.

Die EID₅₀-Titer des nach diesem Verfahren gezüchteten und isolierten menschlichen Influenzavirus-Stamms A/USSR/90/77 sind in Tabelle 5 genannt. Ferner wird auf Fig. 5 verwiesen. Ein Vergleich wird mit diesem nach dem gleichen Verfahren, jedoch ohne Zusatz von Trypsin gezüchteten Stamms vorgenommen.

**Tabelle 5**

| EID₅₀-Titer des in CLDK-Zellen mit und ohne Trypsin gezüchteten menschlichen Influenzavirus, Stamm A/USSR/90/77 | | | |
|---|---|---|---|
| Trypsinmenge, µg/ml | Titer (EID₅₀/ml) Einsatz | nach Isolierung | x-fache Steigerung mit Trypsin |
| 0 | 10^{4,0} | 10^{6,3} | - |
| 10 | 10^{4,0} | 10^{8,7} | 251 |
| 10 | 10^{4,0} | 10^{9,0} | 501 |
| 10 | 10^{4,5} | 10^{8,4} | 126 |

Die Zugabe von Trypsin zum Kulturmedium ergab einen geometrischen mittleren Anstieg von 2,4 log oder 251mal so viele Viruspartikel/ml während der Produktion des A/USSR-Stamms.

### Impfstoffherstellung

### Beispiel 6

### Virusabschwächung

Die Abschwächung des Virus aus den gemäß Beispiel 1 isolierten Flüssigkeiten erfolgt chemisch oder durch Standard-Reihenpassagen einschließlich abschließender Verdünnungspassagenmethoden, bei denen eine genügende Anzahl von Passagen in einer empfänglichen Zellkultur angewendet wird, bis das Virus ohne Verlust der Immunisierungsfähigkeit nicht-pathogen gemacht worden ist. Ein in dieser Weise hergestellter Impfstoff löst bei Tieren, die für die Krankheit anfällig sind, eine Immunreaktion aus, ohne die klinischen Symptone, die normalerweise auf das virulente Mittel zurückzuführen sind, in wesentlichem Ausmaß hervorzubringen. Die Vermehrung kann in den gleichen Geweben, wie sie bei der vorhergehenden Passage verwendet wurden, oder in verschiedenen Geweben erfolgen.

### Beispiel 7

### Inaktivierung des Virus

Die Methode ist ähnlich wie die in den Beispielen 1 und 2 beschriebenen, jedoch werden die isolierten, virusbeladenen Flüssigkeiten durch Inaktivierung mit 0,1%igem Formaldehyd (Bereich 0,05 bis 0,2%) weiterverarbeitet, und das behandelte Material wird 10 bis 14 Tage bei 4°C inkubiert. Die Testung des endgültigen inaktivierten Viruspräparats zeigte, daß es frei von lebendem Virus war. Bekannte Adjuvantien, z. B. Aluminiumhydroxid, Alaun, Aluminiumphosphat, Freund' Adjuvans oder die in den US-PS 3 790 665 und 3 919 411 beschriebenen Adjuvantien können zugesetzt werden. Das bevorzugte Adjuvans bei diesen Veröffentlichungen und in den Impfstoffen gemäß der Erfindung ist ein Acrylsäurepolymerisat, das mit einem Polyallylsaccharid (Carbopol 934 P) ähnlich dem in den vorstehend genannten Patentschriften beschriebenen vernetzt ist.

### Beispiel 8

### Inaktivierter Virus-Impfstoff, Herstellung und Verwendung

Eine Pferdeimpfstoffdosis von 1,0 ml besteht aus 0,45 ml des Stamms Pennsylvania (AL), 0,45 ml des Stamms Miami (A2) und 0,10 ml des vernetzten Acrylsäurepolymerisats (Carbopol 934 P) als Adjuvans. Gleiche Teile der gemäß Beispiel 7 erhaltenen inaktivierten Vaccinestämme wurden gemischt, und aliquote Teile von 1 ml wurden 19 Pferden intramuskulär verabreicht. Keine klinische Krankheit oder Symptome von Influenza wurden bei den Pferden nach der Impfung festgestellt. Die Antikörper-Titer sowohl gegen Pferde-Influenza A1 als auch Pferde-Influenza A2 wurden an den Blutseren aller Tiere nach 2,4 und 8 Wochen nach der Impfung ermittelt. Diese Titer werden mit den Titern vor der Impfung mit Hilfe des Standard-Hämagglutinationshemmungstests (Diagnostic Procedures for Viral and Rikkettsial Infections, 4. Auflage, Lennette und Schmidt, S. 665-666 (1969), American Public Health Association, New York, N. Y. 10 019, verglichen (Tabelle 6).

**Tabelle 6**

| Pferd Nr. | Antikörperreaktionen (Hämagglutinationshemmung) | | | | | | | |
|---|---|---|---|---|---|---|---|---|
| | Pferde-Influenza A1 | | | | Pferde-Influenza A2 | | | |
| | Vorher | 2W. | 4W.*) | 8W. | Vorher | 2W. | 4W.*) | 8 Wochen |
| 2 | <8 | 8192 | 8192 | 8192 | <8 | 512 | 128 | 256 |
| 11 | <8 | 128 | 256 | 128 | <8 | 64 | 1024 | 64 |
| 13 | <8 | 128 | 256 | 2048 | <8 | 256 | 256 | 1024 |
| 19 | <8 | 256 | 64 | 64 | <8 | 128 | 128 | 64 |
| 21 | <8 | 128 | 64 | 512 | <8 | 64 | 64 | 512 |
| 23 | <8 | 256 | 128 | 128 | <8 | 512 | 512 | 128 |
| 29 | <8 | 128 | 32 | 1024 | <8 | 256 | 64 | 128 |
| 32 | <8 | 1024 | 256 | 2048 | <8 | 64 | 64 | 128 |
| 37 | <8 | 128 | 32 | 512 | <8 | 64 | 32 | 64 |
| 38 | <8 | 1024 | 512 | 1024 | <8 | 512 | 256 | 64 |
| 40 | <8 | 256 | 128 | 1024 | <8 | 128 | 128 | 128 |
| 55 | <8 | 512 | 512 | 8192 | <8 | 512 | 256 | 256 |
| 61 | <8 | 512 | 64 | 256 | <8 | 1024 | 128 | 128 |
| 68 | <8 | 128 | 32 | 128 | <8 | 512 | 256 | 256 |
| 79 | <8 | 64 | 128 | 2048 | <8 | 256 | 128 | 128 |
| 121 | <8 | <8 | 32 | 512 | <8 | 1024 | 256 | 64 |
| 125 | <8 | 256 | 32 | 128 | <8 | 256 | 128 | 128 |
| 128 | <8 | 512 | 128 | 512 | <8 | 256 | 128 | 256 |
| 129 | <8 | 256 | 128 | 2048 | <8 | 512 | 512 | 256 |

| | | | | | | | | |
|---|---|---|---|---|---|---|---|---|
| *) Tag des Booster-Effekts | | | | | | | | |

Wie die Werte in Tabelle 6 zeigen, bildeten sich Antikörper gegen beide Virus-Antigene in Pferden, die geimpft wurden. Diese geimpften Tiere würden immun gegen Pferdeinfluenza sein, da Antikörpertiter über 1 : 20 für A2 und 1 : 60 für A1 vom National Veterinary Services Laboratories des USA-Landwirtschaftsministeriums als schützende Titer akzeptiert sind.

Klinische Versuche an 420 Pferden verschiedener Rassen und verschiedenen Alters ergaben, daß der Impfstoff sicher ist und nach intramuskulärer Impfung keine ungünstigen Reaktionen hervorruft.

### Beispiel 9

### Verwendung von abgeschwächtem Virus-Impfstoff

Drei Pferde erhielten intranasal 5 ml der gemäß Beispiel 6 hergestellten Lebendvaccine auf Basis des abgeschwächten Pferde-Influenza-A2-Vaccinestamms. Keine klinische Krankheit oder Symptome von Influenza wurden bei den Pferden nach der Impfung festgestellt. Die Antikörpertiter gegen Vaccinestamm wurden an Blutseren aller Tiere 1,2 und 4 Wochen nach der Impfung ermittelt und mit dem Titer vor der Impfung mit Hilfe des Standard-Hämagglutinationshemmungstests (HAI) verglichen.

**Tabelle 7**

| Antikörperreaktion (HAI) - Pferdeinfluenza A2 | | | | |
|---|---|---|---|---|
| Pferd Nr. | Vorher | 1 Woche | 2 Wochen | 4 Wochen |
| 19 | <8 | 128 | 256 | 128 |
| 23 | 8 | 64 | 128 | 128 |
| 61 | <8 | 128 | 256 | 128 |

Auch hier waren die ermittelten Antikörpertiter höher als für den Schutz erforderlich.

Für das erfindungsgemäß verwendete flüssige Zellkultursystem werden anfällige Zellen, Influenzaviren, ein Nährmedium und ein proteolytisches Enzym verwendet, jedoch erfordert dieses Kultursystem im Gegensatz zu den bekannten Systemen (z. B. das System gemäß Tobita et al) nicht die Verwendung von Agar, das das System in einen halbfesten Zustand bringt. Der Ausschluß von Agar ermöglicht somit die Großerzeugung von Viren in üblicher bekannter Weise und führt zur Erzeugung von viralen Flüssigkeiten mit genügend hohen Titern für die Herstellung von Impfstoffen.

Das Influenza-Impfstoffpräparat selbst enthält wirksame Mengen eines oder mehrerer Stämme von gegebenen Influenza-Viruspartikeln und einen pharmazeutisch unbedenklichen Träger. Das gesamte Präparat, das vorzugsweise in wäßriger Form vorliegt, ist im wesentlichen frei von reaktionsfähigen Proteinen, z. B. Eiereiweiß. Der hier gebrauchte Ausdruck »im wesentlichen frei von Eiereiweiß« bedeutet, daß die einzige mögliche Quelle von Eiereiweiß in den Impfstoffpräparaten das Impfvirus ist das > 1 : 100 000 verdünnt ist.

Die Impfstoffe werden Tieren auf verschiedenen Wegen einschließlich intramuskulär, intravenös, subkutan, intratracheal, intranasal oder durch Aerosolspray verabreicht und sind für die vorteilhafte Verwendung bei den verschiedensten Tieren einschließlich Mensch, Pferd, Schwein und Vögel vorgesehen.

Die gemäß der Erfindung hergestellten Impfstoffe können zur Einstellung ihrer Wirksamkeit mit Wasser verdünnt werden. Ferner können ihnen Stabilisatoren wie Saccharose, Dextrose, Lactose oder andere ungiftige Stoffe zugesetzt werden. Die Viruspräparate können durch Gefriertrocknung für Lagerzwecke oder für die spätere Formulierung zu abgeschwächten Vaccinen getrocknet werden, oder sie können für die Herstellung von Virus-Tot-Impfstoffen chemisch inaktiviert werden.

## Patentansprüche

1. Verfahren zur Herstellung einer Influenzavirus-Vaccine, die in wesentlichen frei von Eiereiweiß ist, wobei man Zellen mit einem Influenzavirus infiziert, das Virus in den Zellen vermehrt, danach das Virus isoliert und aus dem isolierten Virus eine Vaccine herstellt, dadurch gekennzeichnet, daß man einen Teil der Zellen einer flüssigen Zellkultur mit einem Influenzavirus infiziert und die infizierte Kultur in Gegenwart von 4 - 25 µg/ml der flüssigen Zellkultur eines proteolytischen Enzyms inkubiert.

2. Verfahren nach Anspruch 1, dadurch gekennzeichnet, daß als proteolytisches Enzym Trypsin, Chymotrypsin, Pepsin, Pancreatin, Papain, Pronase oder Carboxypeptidase eingesetzt werden.

3. Verfahren nach Anspruch 1 und 2, dadurch gekennzeichnet, daß als proteolytisches Enzym Trypsin in einer Menge von 8 - 15 µg/ml der flüssigen Zellkultur zugesetzt wird.

4. Verfahren gemäß Anspruch 1 bis 3, dadurch gekennzeichnet, daß die Kultur in Rollflaschen durchgeführt wird.

5. Verfahren gemäß Anspruch 1 bis 4, dadurch gekennzeichnet, daß proteolytisches Enzym zur zuvor infizierten Kultur zugegeben wird.

6. Verfahren nach Anspruch 1 bis 5, dadurch gekennzeichnet, daß als Virus ein Pferde-Influenzavirus oder ein menschliches Influenzavirus eingesetzt werden.

7. Verfahren nach Anspruch 1 bis 6, dadurch gekennzeichnet, daß man als Zellen in der flüssigen Zellkultur Hundenierenzellen verwendet.

8. Influenza-Vaccine, erhältlich nach dem Verfahren gemäß Anspruch 1 bis 7.

9. Influenza-Vaccinepräparat gemäß Anspruch 8, gekennzeichnet durch eine wirksame Menge Influenzaviruspartikel und einen pharmazeutisch unbedenklichen Träger, wobei das Präparat frei von Eiereiweiß ist

## Claims

1. Process for the preparation of an influenza virus vaccine which is essentially free of egg albumin, in which cells are infected with an influenza virus, the virus is replicated in the cells, then the virus is isolated and a vaccine is prepared from the isolated virus, characterized in that a part of the cells of a liquid cell culture are infected with an influenza virus and the infected culture is incubated in the presence of 4-25µg/ml of the liquid cell culture of a proteolytic enzyme.

2. Process according to Claim 1, characterized in that, as proteolytic enzyme, trypsin, chymotrypsin, pepsin, pancreatin, papain, pronase or carboxypeptidase is employed.

3. Process according to Claims 1 and 2, characterized in that, as proteolytic enzyme, trypsin is added in an amount of 8-15µg/ml of the liquid cell culture.

4. Process according to Claims 1 to 3, characterized in that the culture is carried out in narrow-necked cylindrical bottles.

5. Process according to Claims 1 to 4, characterized in that proteolytic enzyme is added to the previously infected culture.

6. Process according to Claims 1 to 5, characterized in that, as virus, an equine influenza virus or a human influenza virus is employed.

7. Process according to Claims 1 to 6, characterized in that, as cells in the liquid cell culture, canine kidney cells are used.

8. Influenza vaccine, obtainable by the process according to Claims 1 to 7.

9. Influenza vaccine preparation according to Claim 8, characterized by an efficacious amount of influenza virus particles and a pharmaceutically acceptable excipient, the preparation being free of egg albumin.

## Revendications

1. Procédé de préparation d'un vaccin contre le virus de la grippe, qui est essentiellement dépourvu d'albumine d'oeuf, dans lequel on infecte des cellules avec un virus de la grippe, on multiplie le virus dans les cellules, puis on isole le virus et on prépare un vaccin à partir du virus isolé, caractérisé en ce qu'on infecte une partie des cellules d'une culture liquide avec un virus de la grippe et on fait incuber la culture infectée en présence de 4 à 25 µg, par ml de culture liquide de cellules, d'un enzyme protéolytique.

2. Procédé suivant la revendication 1, caractérisé en ce qu'on utilise comme enzyme protéolytique la trypsine, la chymotrypsine, la pepsine, la pancréatine, la papaïne, la pronase ou la carboxypeptidase.

3. Procédé suivant les revendications 1 et 2, caractérisé en ce qu'on ajoute de la trypsine comme enzyme protéolytique à la culture liquide de cellules en une quantité de 8 à 15 µg/ml.

4. Procédé suivant les revendications 1 à 3, caractérisé en ce que la culture est conduite dans des flacons agités par roulement.

5. Procédé suivant les revendications 1 à 4, caractérisé en ce que l'enzyme protéolytique est ajouté à la culture préalablement infectée.

6. Procédé suivant les revendications 1 à 5, caractérisé en ce qu'on utilise comme virus un virus grippal équin ou un virus grippal humain.

7. Procédé suivant les revendications 1 à 6, caractérisé en ce qu'on utilise comme cellules des cellules rénales de chien dans la culture liquide de cellules.

8. Vaccin contre la grippe, pouvant être obtenu par le procédé suivant les revendications 1 à 7.

9. Préparation de vaccin contre la grippe suivant la revendication 8, caractérisée par une quantité efficace de particules virales grippales et un support pharmaceutiquement acceptable, la préparation étant dépourvue d'albumine d'oeuf.
